# EUROPEAN PATENT SPECIFICATION

(11) **EP 0 435 497 B1**
(45) Date of publication and mention of the grant of the patent: **16.02.1994**
(21) Application number: 90313336.1
(22) Date of filing: 07.12.1990
(51) Int. Cl.: C07C 237/04, C08G 59/50

(54) **Trifunctional polyoxyethylene diamine derivatives**
Trifunktionelle Polyoxyethylenediaminderivate
Dérivés de polyoxyéthylènes diamines trifonctionnelles

(30) Priority: 26.12.1989 US 456892
(43) Date of publication of application: 03.07.1991
(73) Proprietor: TEXACO CHEMICAL COMPANY, Houston, Texas 77056 (US)
(72) Inventor: Speranza, George Phillip, Sr., Austin, Texas 78757 (US); Lin, Jiang-Jen, Houston, Texas 77083 (US)
(74) Representative: Brock, Peter William

(56) References cited:
- EP-A- 0 174 725
- US-A- 3 175 987
- US-A- 4 495 366
- US-A- 4 517 378

## Description

This invention relates to normally liquid trifunctional derivatives obtained by reaction of polyoxyethylene diamines with an unsaturated dicarboxylic acid component which is preferably a dialkyl maleate. More particularly, this invention relates to normally liquid trifunctional derivatives that are useful, for example, as curing agents for epoxy resins.

US-A-4495366 discloses the preparation of trifunctional derivatives of diamines by the reaction of a diamine with an unsaturated dicarboxylic acid or ester thereof, such as diethyl maleate. The diamines that are used are polymethylene diamines having the formula:

H₂N(CH₂)ₙNH₂

wherein n is 4 to 8.

The trifunctional derivatives formed in this manner are solid at room temperature, however, and therefore are preferably prepared by conducting the reaction in the presence of a solvent, and by using the trifunctional derivative while in solution in a suitable solvent such as an aromatic hydrocarbon (e.g. toluene).

US-A-4572870 is directed to the preparation of crosslinking agents for chlorosulfonated ethylene/vinyl acetate polymer coating compositions by reacting 3 moles of an aliphatic or cycloaliphatic polyamine with 1 mole of a dialkyl maleate at 100 to 150°C for 1 to 6 hours. The coating compositions comprise 20 to 90 wt.% of an organic solvent, and 10 to 80 wt.% of a binder, comprising a chlorosulfonated ethylene/vinyl acetate polymer, an epoxy resin, and a polyamine curing agent having the formula: 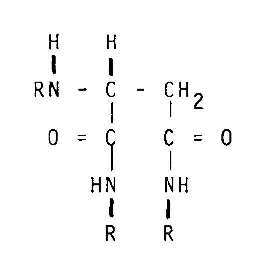
wherein R is an aliphatic or cycloaliphatic hydrocarbon group.

The coating composition also contains a bicyclic amidine.

US-A-4579674 is directed to hydrocarbylsuccinimides of secondary hydroxyl-substituted polyamines, and lubricating oils containing them, and US-A-4579675 is directed to N-substituted enaminones and oleaginous compositions containing them.

The present invention relates to a compound of the formula: 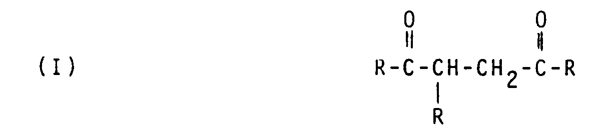
wherein each R is a group of the formula: 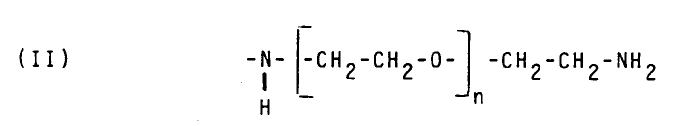
in which n is 2 to 3.

The present invention also relates to the use of these compounds as curing agents for epoxy resins.

The present invention also relates to a method of making a compound as defined above, which comprises reacting a carboxylic acid component selected from maleic acid, fumaric acid, anhydrides thereof and C₁ to C₄ dialkyl maleates and fumarates, with 3 to 3.5 moles, per mole of said carboxylic acid component, of a polyoxyethylene diamine of the formula: 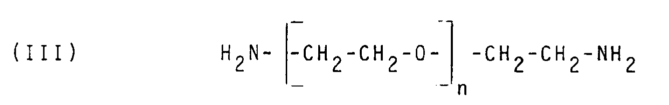
wherein n is 2 to 3.

The reaction involves both Michael addition of the polyoxyalkylene diamine at the double bond of the unsaturated dicarboxylic acid component, and amide formation, by reaction of the carboxyl groups of the unsaturated dicarboxylic acid with amine groups of the polyoxyethylene diamine.

The trifunctional derivatives of polyoxyethylene diamines and unsaturated dicarboxylic acids obtained in accordance with the present invention are useful as curing agents for epoxy resins. They have many desirable properties, for example, they are liquid at ambient temperatures and therefore may be mixed with epoxy resins with ease and without the use of solvents. The cured epoxy resins formed with the trifunctional polyoxyethylene diamine derivatives of the present invention will be of ameliorated hardness because of the presence of the polyoxyethylene groups which are, in effect, internal plasticizers.

The diamine component and the unsaturated carboxylic acid component are heated in a suitable reaction vessel, such as an autoclave provided with agitation means and means for regulating temperature, so that the reaction can be conducted at ambient pressure at temperatures within the range of about 30 to 170°C with agitation for a period of time within the range of about 2 to about 10 hours sufficient to permit by-product water and alcohols to evaporate from the reaction mixture. Consequently, the resultant reaction product will consist essentially of the desired trifunctional derivative of the present invention.

The starting materials for the present invention are a polyoxyethylene diamine component and an unsaturated dicarboxylic acid component.

The polyoxyethylene diamine component is a diamine having the formula: 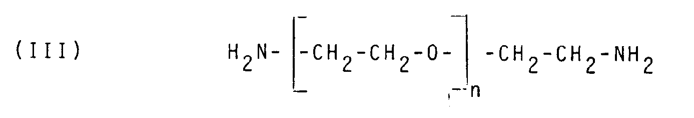
wherein n is 2 or 3.

For example, when n is 2, the resultant polyoxyethylene diamine will have a molecular weight of about 148. A product of this nature is sold by Texaco Chemical Company under the tradename Jeffamine^{R} EDR-148 amine.

When n is 3, the resultant polyoxyethylene diamine will have a molecular weight of about 192 and is sold commercially by Texaco Chemical Company as the product identified as Jeffamine^{R} EDR-192 amine.

The unsaturated dicarboxylic acid component is maleic acid or fumaric acid, an anhydride thereof or a C₁ to C₄ alkyl ester thereof. Preferred starting materials are the dialkyl maleates, such as dimethyl maleate, diethyl maleate, ethylmethyl maleate, dipropyl maleate and dibutyl maleate.

The reaction is conducted at a temperature of 30 to 170°C for a period of 2 to 10 hours, sufficient to permit by-product water and alkyl alcohols to evaporate from the reaction mixture.

Thus, for example, if about 3 moles of a polyoxyethylene diamine having a molecular weight of about 148 are reacted with one mole of dimethyl maleate, the reaction sequence may be schematically illustrated by the following equation, wherein one mole of the polyoxyethylene diamine reacts with the dimethyl maleate by Michael addition and 2 moles of the polyoxyethylene diamine react with the diethyl maleate to form amides, and 2 moles of methanol are formed as a by-product.

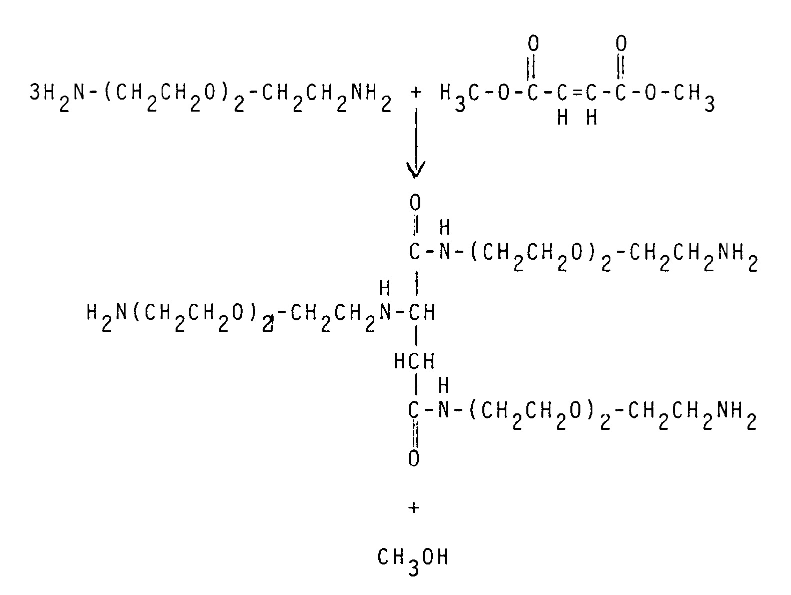

As seen from the above equation, 3 moles of the polyoxyethylene diamine will react with each mole of the unsaturated dicarboxylic acid component. However, it is desirable to use a slight excess of the diamine and, accordingly, in practice, from 3 to 3.5 moles of the polyoxyethylene diamine will be used for each mole of the unsaturated dicarboxylic acid component.

The trifunctional derivatives of the polyoxyethylene diamines prepared in accordance with the present invention are useful as curing agents for epoxy resins. A curable epoxy resin composition is prepared by mixing an epoxide with the trifunctional polyoxyethylene diamine derivative of the present invention, in an amount such that the ratio of active amino hydrogens to epoxy groups in the mixture is from 0.5 to 2:1, and more preferably from 0.8 to 1.5:1.

The curable epoxy resin compositions are generally vicinal epoxide compositions having an average of more than one reactive 1,2-epoxide group per molecule.

These epoxide materials can be monomeric or polymeric, saturated or unsaturated, aliphatic, cycloaliphatic, aromatic or heterocyclic, and may be substituted if desired with other substituents besides the epoxy groups, e.g. hydroxyl groups, ether radicals or halogenated phenyl groups.

The most widely used epoxy resins are diglycidyl ethers of bisphenol A: 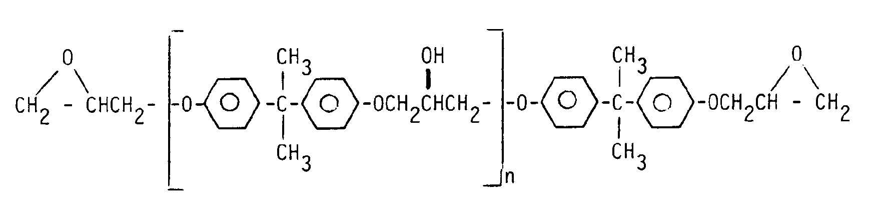
wherein n is 0 to 5.

These epoxides, however, are representative of the broader class of epoxide compounds that are useful in making epoxy resins.

A widely used class of polyepoxides that can be cured according to the practice of the present invention includes the resinous epoxy polyethers obtained by reacting an epihalohydrin, such as epichlorohydrin, with either a polyhydric phenol or a polyhydric alcohol. An illustrative, but by no means exhaustive, listing of suitable dihydric phenols includes 4,4'-isopropylidene bisphenol, 2,4'-dihydroxydiphenylethylmethane, 3,3'-dihydroxydiphenyldiethylmethane, 3,4'-dihydroxydiphenylmethylpropylmethane, 2,3'-dihydroxydiphenylethylphenylmethane, 4,4'-dihydroxydiphenylmethane, 4,4'-dihydroxydiphenylbutylphenylmethane, 2,2'-dihydroxydiphenylditolylmethane, and 4,4'-dihydroxydiphenyltolylmethylmethane. Other polyhydric phenols which may also be co-reacted with an epihalohydrin to provide these epoxy polyethers are such compounds as resorcinol, hydroquinone, and substituted hydroquinones, e.g. tertbutylhydroquinone.

The following specific examples are given by way of illustration of the products and process of the present invention. Where parts are mentioned, they are parts by weight.

### Example 1 - Adduct of Diethyl Maleate and EDR-192 (1:3 molar ratio)

To a 500 ml three-necked flask equipped with a thermometer, Dean-Stark trap, mechanical stirrer and nitrogen inlet line, was charged Jeffamine^{R} EDR-192 amine (tetraethylene glycol diamine, 204g, 94% purity, 4% water, ca. 1.0M). While stirring at room temperature, diethyl maleate (57g, ca. 0.33M) was added slowly, in several portions. The reaction occurred exothermically at 25 to 60°C. The mixture was heated to 150°C. At this temperature, ethanol was generated and removed through the Dean-Stark trap. After four hours, the mixture was cooled to room temperature. The reaction product was a viscous yellow liquid with a viscosity of 118 x 10⁻³ m²/s at 25°C (1918 cs/25°C). The amine content was 5.93 meq/g (calc. 6.2 meq/g). The analyses of H-nmr and IR suggested the complete reaction of Michael additions and amide formation. The major product was described to be:

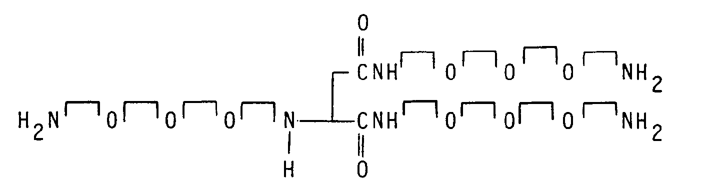

### Example 2 - Adduct of Diethyl Maleate and EDR-148 (1:3 molar ratio)

To a 500 ml three-necked flask equipped with a thermometer, Dean-Stark trap, stirrer and nitrogen inlet line, was charged Jeffamine^{R} EDR-148 amine (148g, 1M). While stirring, diethyl maleate (57g 0.333M) was added in several portions over 20 minutes. The maximum exothermic temperature was 50°C. The mixture was heated to 150 - 160°C. Ethanol was generated, and removed at room temperature. The product was a viscous, yellow liquid, having a viscosity of 3.259 x 10 ⁻³ m²/s at 25°C (3259 cs/25°C). The IR exhibited peaks at 1670 cm⁻¹ 1550 cm⁻¹ (amide), 1130 cm⁻¹ (ether), and very small ester absorption at 1750 cm⁻¹ . The amine content was 7.68 meq/g (calc. 7.6 meq/g). In addition, H-nmr showed no olefin functional group. These analyses suggested the product was a 3:1 adduct as follows:

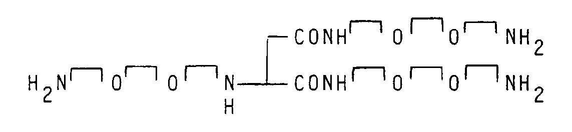

### Example 3 - Adduct of Diethyl Maleate and Tripropylene Glycol Diamine (1:3 molar ratio) (Comparative Example)

To a 500 ml three-necked flask equipped with a thermometer, Dean-Stark trap, stirrer and nitrogen line was charged tripropylene glycol diamine (171g, 0.9M). Diethyl maleate (64.5g, 0.3M) was added with stirring. The mixture was heated at 150 - 170°C over three hours. No ethanol was generated during the process. After cooling to room temperature, the product was analyzed by H-nmr, indicating that the desired product was not obtained.

### Example 4 - Adduct of Dimethyl Maleate and EDR-192 (1:3 molar ratio)

Following the procedures of Example 1, except for using dimethyl maleate instead of diethyl maleate, the adduct of maleate/EDR-192 at 1:3 molar ratio was made.

### Example 5 - Adduct of Dimethyl Maleate and EDR-148 (1:3 molar ratio)

Following the procedure of Example 1, the adduct of dimethyl maleate and EDR-148 (1:3 molar ratio) was made.

### Example 6 - Adduct of Diethyl Maleate and EDR-192 (0.6:1 molar ratio) (Comparative Example)

To a 500 ml three-necked flask equipped with a thermometer, Dean-Stark trap, stirrer and nitrogen inlet line, was charged EDR-192 (192g, 1M). With stirring, diethyl maleate (108g, 0.62M) was added. The mixture was heated to 140°C for one hour. A gel-like material was obtained.

### Example 7 - Adduct of Maleate/EDR-148 (1:2.4 molar ratio) (Comparative Example)

To a 500 ml three-necked flask equipped with a thermometer, Dean-Stark trap, stirrer and nitrogen inlet line, was charged EDR-148 (133g, 0.9M). With stirring, diethyl maleate (64.5g, 0.375M) was added slowly. The exothermic temperature at 45°C was observed. The mixture was heated t 145 to 149°C for six hours to remove ethanol. A brown, viscous liquid was obtained. The analysis showed 6.55 meq/g amine content. Upon further standing in an oven over a long period, the material turned into a gel-like product. A ratio of at least 1:3 was required for maleate/ EDR-148 to form a non-crosslinking product.

## Claims

1. A compound of the formula: 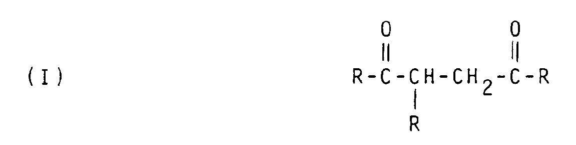 wherein each R is a group of the formula: 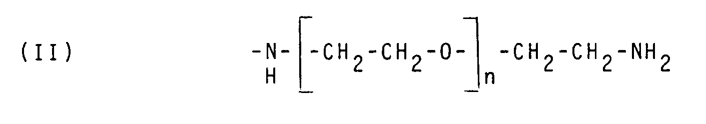 in which n is 2 to 3.

2. A compound according to Claim 1 characterized in that n is 2.

3. A compound according to Claim 1 characterized in that n is 3.

4. A method of making a compound according to any one of Claims 1 to 3 which comprises reacting a carboxylic acid component selected from maleic acid, fumaric acid, anhydrides thereof and C₁ to C₄ dialkyl maleates and fumarates, with 3 to 3.5 moles, per mole of said carboxylic acid component, of a polyoxyethylene diamine of the formula: 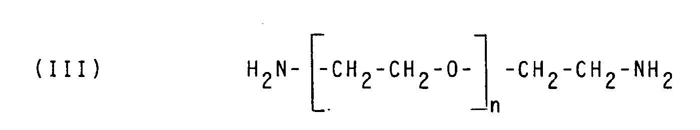 wherein n is 2 to 3, said reaction being conducted in the absence of a solvent at a temperature of 30 to 170°C with agitation for 2 to 10 hours.

5. A method according to Claim 4 characterized in that the carboxylic acid component is a C₁ to C₄ alkyl maleate.

6. A method according to Claim 5 characterized in that the maleate is dimethyl maleate.

7. A method according to Claim 5 characterized in that the maleate is diethyl maleate.

8. Use of a compound according to any one of Claims 1 to 3 as a curing agent for epoxy resins.

## Patentansprüche

1. Verbindung der Formel: 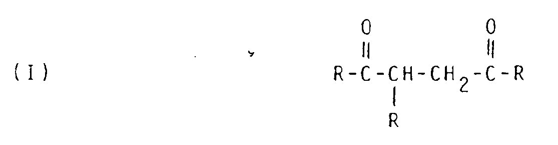 wobei jedes R eine Gruppe der Formel ist: 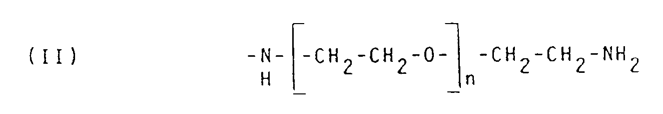 in der n 2 bis 3 ist.

2. Verbindung nach Anspruch 1, dadurch gekennzeichnet, daß n 2 ist.

3. Verbindung nach Anspruch 1, dadurch gekennzeichnet, daß n 3 ist.

4. Verfahren zur Herstellung einer Verbindung nach einem der Ansprüche 1 bis 3, welches umfaßt, daß eine Carbonsäure-Komponente, die ausgewählt ist aus Maleinsäure, Fumarsäure, Anhydriden derselben und C₁-C₄-Dialkylmaleaten und -fumaraten, mit 3 bis 3,5 Mol, pro Mol besagter Carbonsäure-Komponente, eines Polyoxyethylendiamins der Formel: 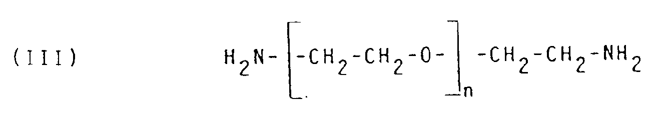 in der n 2 bis 3 ist, zur Reaktion gebracht wird, wobei besagte Reaktion in der Abwesenheit eines Lösungsmittels bei einer Temperatur von 30 bis 170°C mit Bewegung über 2 bis 10 Stunden durchgeführt wird.

5. Verfahren nach Anspruch 4, dadurch gekennzeichnet, daß die Carbonsäure-Komponente ein C₁-C₄-Alkylmaleat ist.

6. Verfahren nach Anspruch 5, dadurch gekenzeichnet, daß das Maleat Dimethylmaleat ist.

7. Verfahren nach Anspruch 5, dadurch gekennzeichnet, daß das Maleat Diethylmaleat ist.

8. Verwendung einer Verbindung nach einem der Ansprüche 1 bis 3, als ein Härtungsmittel für Epoxyharze.

## Revendications

1. Composé de formule : 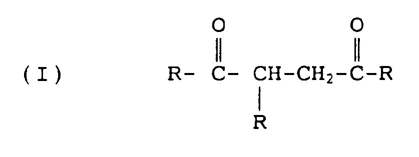 où chaque R est un groupe de formule : 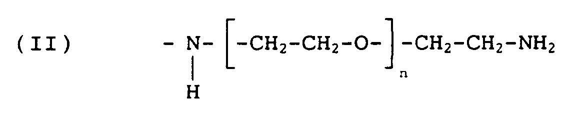 où n est 2 à 3.

2. Composé selon la revendication 1, caractérisé en ce que n est 2.

3. Composé selon la revendication 1, caractérisé en ce que n est 3.

4. Procédé de réalisation d'un composé selon l'une quelconque des revendications 1 à 3, qui comprend la réaction d'un composant d'acide carboxylique choisi parmi l'acide maléique, l'acide fumarique, les anhydrides de ceux-ci, et des maléates et fumarates de dialkyle C₁ à C₄, avec 3 à 3,5 moles, par mole dudit composant d'acide carboxylique, d'une polyoxyéthylènediamine de formule : 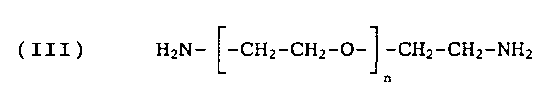 où n est 2 à 3, ladite réaction étant réalisée en l'absence d'un solvant à une température de 30 à 170 °C sous agitation pendant 2 à 10 heures.

5. Procédé selon la revendication 4, caractérisé en ce que le composant d'acide carboxylique est un maléate d'alkyle C₁ à C₄.

6. Procédé selon la revendication 5, caractérisé en ce que le maléate est le diméthylmaléate.

7. Procédé selon la revendication 5, caractérisé en ce que le maléate est le diéthylmaléate.

8. Utilisation d'un composé selon l'une quelconque des revendications 1 à 3 comme agent de durcissement pour résines époxy.
